# EUROPÄISCHE PATENTSCHRIFT

(11) **EP 0 836 846 B1**
(45) Veröffentlichungstag und Bekanntmachung des Hinweises auf die Patenterteilung: **09.08.2000**
(21) Anmeldenummer: 97118203.5
(22) Anmeldetag: 21.10.1997
(51) Int. Cl.: A61K 7/00

(54) **Mine für Farbstifte, Kosmetikstifte und Farbkreiden sowie Verfahren zu ihrer Herstellung**
Pencil lead for colour pencils, cosmetic pencils and colour chalks and process of manufacturing same
Mine de crayon pour crayons à colorier, crayons cosmétiques et craies à colorier et son procédé de fabrication

(30) Priorität: 21.10.1996 DE 19643356
(43) Veröffentlichungstag der Anmeldung: 22.04.1998
(73) Patentinhaber: A.W. Faber- Castell Unternehmensverwaltung GmbH & Co., D-90547 Stein (DE)
(72) Erfinder: Lugert, Gerhard, Dr., 90431 Nürnberg (DE)
(74) Vertreter: Tergau, Enno, Dipl.-Ing.

(56) Entgegenhaltungen:
- EP-A- 0 697 447
- DE-A- 4 230 793
- US-A- 5 360 281

## Beschreibung

Die Erfindung betrifft eine Mine für Farbstifte, Kosmetikstifte und Farbkreiden sowie ein Verfahren zu ihrer Herstellung. Minen dieser Art enthalten in der Regel ein Bindemittel, beispielsweise ein Cellulosederivat, Füllstoffe wie Kaolin und Farbmittel. Zur Herstellung werden die Ausgangsstoffe mit Wasser, beispielsweise in einem Kneter miteinander zu einer Minenmasse vermischt. Aus dieser Minenmasse werden Rohminen extrudiert und bei Temperaturen bis etwa 110° C getrocknet. Die Konsistenz bzw. Härte einer Mine wird entscheidend durch das verwendete Bindemittel und dessen Konzentration bestimmt. Darüber hinaus hängt die mechanische Stabilität und die Konsistenz einer Mine von den Zusatzstoffen, insbesondere auch von den Füllstoffen ab EP-A-0 697 447 beschriebt ein Verfahren zur Herstellung einer bestimmen Art von Minen, wobei die aus einem polymeren Bindemittel und anorganischen Füllstoff bestehende Minenmasse erhitzt wird, um den organischen Binder zu zersetzen und aus der Mine vollständig zu entfernen. Um das Abstrichverhalten bei herkömmlichen Minen zu verbessern, werden diese nach dem Trocknen in flüssiges Wachs, flüssige Fette oder Fettsäuren getaucht. Um das Abstrichverhalten der Minen merklich in Richtung auf einen weicheren Abstrich zu verändern, muß eine Wachsaufnahme zwischen etwa 15% und 25% des Gesamtgewichts der Mine erfolgen. Eine andere Möglichkeit, das Abstrichverhalten der Rohminen zu verbessern, besteht darin, bereits den Ausgangsstoffen Fette, Wachse, Fettsäuren oder auch Emulgatoren beizumengen. Das verbesserte Abstrichverhalten wird also stets mit einem relativ hohen Anteil an Fetten, Wachsen, Fettsäuren oder Emulgatoren erkauft. Für manche Anwendung ist jedoch ein solch hoher Anteil an den genannten Stoffen nicht gewünscht. Eine weitere Möglichkeit, das Abstrichverhalten zu beeinflussen, besteht darin, den Bindemittelanteil zu reduzieren. Damit ist aber einer Verringerung der mechanischen Stabilität der Rohminen verbunden. Bevor die bindemittelarmen Rohminen aber ihre endgültige Festigkeit erlangt haben besteht erhöhte Bruchgefahr. Gesteigerte Ausschußraten durch Minenbruch sind die Folge.

Davon ausgehend ist es die Aufgabe der Erfindung, eine Mine für Farbstifte, Kosmetikstifte und Farbkreiden mit verbessertem und in herstellungstechnischer Sicht einfacher variierbarem Abstrichverhalten sowie ein Verfahren zu ihrer Herstellung vorzuschlagen.

Diese Aufgabe wird durch eine Mine nach Anspruch 1 sowie ein Verfahren nach Anspruch 8 gelöst. Danach enthält die Mine ein durch eine thermische Behandlung teilweise zersetztes polymeres Bindemittel. Neben den bereits oben erwähnten, die Festigkeit und Abstrichverhalten beeinflussenden Faktoren, wie Gewichtsanteil und Art des Bindemittels steht hier eine weitere Möglichkeit zur Beeinflussung insbesondere des Abstrichverhaltens, zur Verfügung. Die die Minen-Inhaltsstoffe zusammenhaltende Bindemittelmatrix kann nämlich durch den Grad ihrer thermischen Zersetzung verändert werden. So können beispielsweise bei Minen mit sonst vergleichbarer Zusammensetzung allein durch Änderung des thermischen Zersetzungsgrades unterschiedliche Konsistenzen und mechanische Stabilitäten sowie ein unterschiedliches Abstrichverhalten erreicht werden. Außerdem stehen Minen zur Verfügung, die trotz des Fehlens des üblichen das Abstrichverhalten bestimmenden hohen Fett- und Wachsanteils, einen weichen Abstrich bei ausreichender Minenmasseabgabe auf die Unterlage zeigen. Zur Herstellung einer erfindungsgemäßen Mine werden die zerkleinerten, etwa pulver- oder granulatförmigen, ein thermolabiles Bindemittel, anorganische Füllstoffe und Farbmittel enthaltenden Ausgangsstoffe mit Wasser vermischt und aus der so erhaltenen Rohmasse Rohminen extrudiert. Die Rohminen werden im Anschluß oder auch später mit einer das Bindemittel teilweise zersetzenden Temperatur behandelt. Die Höhe der Behandlungstemperatur und die Dauer der Wärmebehandlung bestimmen, abhängig vom jeweilig verwendeten Bindemittel, wesentlich die Konsistenz und das Abstrichverhalten der Mine. Bei Minen-Herstellungsverfahren der in Rede stehenden Art ist es, wie bereits oben erwähnt, nicht ohne weiteres möglich, den Bindemittelanteil in der Rohminenmasse zu reduzieren. Durch die mechanische Beeinträchtigung bei der Extrusion der Rohminen sowie bei dem sich anschließenden Weitertransport in eine Trockenstation bzw. eine Wärmebehandlungsstation, hätte ein reduzierter Bindemittelanteil einen erhöhten Minenbruch zur Folge. Dem erfindungsgemäßen Verfahren liegt die Überlegung zugrunde, quasi den Bindemittelanteil in einem abschließenden Verfahrensschritt, in dem die Minen ohne mechanische Beeinflussung einer Wärmebehandlung unterzogen werden, zu reduzieren bzw. das Bindemittel in seiner Wirksamkeit quasi abzuschwächen und damit das Abstrichverhalten der Mine zu verändern. Auf eine Modifizierung des Abstrichverhaltens durch Eintauchen der fertigen Mine in Wachse, Fette, Fettsäuren oder auch Emulgatoren kann ganz verzichtet werden. Eine solche Maßnahme kann aber zweckmäßig sein, wenn die wärmebehandelte Mine in Richtung auf ein weicheres Abstrichverhalten oder aus sonstigen Gründen modifiziert werden soll. Das Fett, das Wachs, die Fettsäure oder der Emulgator kann aber auch bereits der Minenausgangsmischung beigemengt werden. Zweckmäßig dabei ist es, den Gehalt dieser Stoffe auf maximal 2 Gew.% zu beschränken. Größere Mengen werden aufgrund der Verflüssigung dieser Stoffe während der Wärmebehandlung nicht von der Minenmatrix zurückgehalten, können aber durch Eintauchen in die verflüssigten Wachse, Fette oder Fettsäuren nach der Wärmebehandlung eingebracht werden

Vorzugsweise werden als Bindemittel Biopolymere aus der Gruppe der Cellulosederivate und der Alginate eingesetzt. Unter Alginaten sind Salze und Ester der Alginsäure zu verstehen. Solche Bindemittel weisen eine gute Bindefähigkeit für die Mineninhaltsstoffe auf. Ein wesentlicher Vorteil dieser Bindemittel ist jedoch ihre thermische Zersetzbarkeit, wobei das Ausmaß ihrer Zersetzung über die Höhe der Temperatur (im folgenden mit Zersetzungstemperatur bezeichnet) und die Dauer der Wärmebehandlung in feinen Graduierungen einstellbar ist.

Eine bevorzugte Mine enthält (in Gewichtsprozent) 0,5% - 12,5% Bindemittel, 30% - 90% Füllstoffe und 5% - 96% Farbmittel. Gegebenenfalls können Wachse, Fette oder Fettsäuren bis zu 25% und Hilfsstoffe jeweils bis zu 10% vorhanden sein. Der Wasseranteil liegt unterhalb von 1%. Als Füllstoffe werden vorzugsweise natürliche Magnesiumsilikate, Kaolin, fein vermahlenes Quarz- und Bimsmehl, Talkum, vermahlener Ton und Glimmer eingesetzt. Unter Glimmer sind dabei zu den Phyllosilikaten zählende Alkali-, Hydroxyl- und auch fluorhaltige Tonerdesilikate zu verstehen. Als Farbmittel können anorganische oder organische Pigmente, Farbstoffe oder verlackte Farbstoffe sowie beschichtete oder unbeschichtete Metallbronzen verwendet werden. Unter beschichteten Metallbronzen sind solche zu verstehen, bei denen die Metallpartikel einen Überzug beispielsweise aus Siliziumdioxid aufweisen. Als Hilfsstoffe können herkömmliche Tenside, Glykole, Alkohole, Fette oder Fettsäuren, Wachse und Emulgatoren verwendet werden. Die Fette, Wachse und Fettsäuren können der Minen-Ausgangsmischung beigemengt werden. Es ist aber auch denkbar, die erfindungsgemäßen Minen nach ihrer Fertigstellung auf herkömmliche Weise in geschmolzene Wachse, Fette oder Fettsäuren einzutauchen, um ggf. das durch das teilweise zersetzte Bindemittel bewirkte weiche Abstrichverhalten noch zu verbessern. Dazu ist aber ein geringerer Anteil als bei herkömmlichen Minen erforderlich.

Die Wärmebehandlung des erfindungsgemäßen Verfahrens kann abhängig von den verwendeten Ausgangsstoffen der Höhe der Zersetzungstemperatur und dem gewünschten Zersetzungsgrad des Bindemittels bis zu 24 Std. dauern.

Vorzugsweise setzt sich das Bindemittel aus wenigstens einem Stoff zusammen, der ausgewählt ist aus der Gruppe Hydroxyethylcellulose, Methylhydroxyethylcellulose, Ammonium-Carboxymethylcellulose, Alkalicarboxymethylcellulose und Alginate.

Vorzugsweise werden bei einem erfindungsgemäßen Verfahren die Rohminen vor der das Bindemittel teiweise zersetzenden Wärmebehandlung bei einer Trockentemperatur von 30° C bis 110° C getrocknet. Erst nach diesem Trocknungsschritt schließt sich die Wärmebehandlung an. Beide Verfahrensschritte können in ein und demselben Wärmeofen durchgeführt werden, wobei in der Trocknungsphase eine entsprechend niedrige Temperatur eingehalten wird. Bei der Wärmebehandlung hat es sich gezeigt, daß die besten Ergebnisse erhalten werden, wenn die Zersetzungstemperatur im wesentlichen über die gesamte Dauer der Wämebehandlung gleichmäßig gehalten wird. In manchen Fällen kann aber auch eine stufige Temperaturführung zweckmäßig sein, wobei doch in jeder Temperaturstufe die jeweilige Temperatur auf einem gleichmäßigen Niveau gehalten wird.

Die Erfindung wird nun anhand von Beispielen näher erläutert:

### Beispiel 1:

Herstellung einer blauen, zylindrischen Farbmine mit einem Minendurchmesser von 3,8 mm.

Zusammensetzung der Minenmasse:

| Zusammensetzung der Minenmasse: | |
|---|---|
| Natrium-Carboxymethylcellulose | 6,0 g |
| Kaolin | 176,0 g |
| Kupferphthalocyaninblau (Pigment Blue 15 C. I. Nr. 74160) | 18,0 g |

Zur Herstellung werden zunächst die pulverförmigen Bestandteile mit 40 ml Wasser homogen vermischt. Aus der dabei entstandenen Minenmasse werden Minen extrudiert und diese bei 40° C ca. 12 Stunden getrocknet. Im Anschluß daran werden die Rohminen bei einer gleichbleibenden Temperatur von 180° C 20 Minuten lang getempert.

Es werden Minen erhalten, deren Abstrichverhalten gegenüber den Rohminen, deutlich verbessert ist. Die Minen zeigen also eine gute Minenmassenabgabe auf Papier und weisen einen gleitenden weichen Abstrich auf. Die Bruchfestigkeit der Minen wird nur um ca. 10% gegenüber den Rohminen reduziert.

### Beispiel 2:

Herstellung einer weinroten zylindrischen Farbmine mit einem Durchmesser von 4,3 mm.

| Zusammensetzung der Minenmasse: | |
|---|---|
| Methylhydroxyethylcellulose | 4,0 g |
| feinvermahlenes Quarzmehl | 60,0 g |
| Bimsmehl | 60,0 g |
| Talkum | 40,0 g |
| Eisenoxidpigment (Pigment Red 101 CAS Nr.1309-37-1) | 5,0 g |
| Azorot-Pigment (Pigment Red 170 C.I.Nr. 12475) | 6,6 g |
| Ruß (Pigment Black 7 C.I.Nr. 77266) | 0,6 g |
| Stearinsäure | 2,0 g |
| Glyzerin | 2,0 g |
| Zinksulfid-/Bariumsulfatpigment CAS Nr.1345-05-07) | 19,8 g |

Gemäß Beispiel 1 werden zunächst Rohminen hergestellt und diese einer 20-minütigen Mikrowellenbestrahlung von 850 Watt unterzogen.

Der Abstrich der so erhaltenen Mine ist deutlich weicher als der Abstrich der Rohmine. Die Bruchfestigkeit der Mine ist um ca. 15 % gegenüber den ursprünglichen Rohminen verringert.

### Beispiel 3:

Herstellung einer hellblauen zylindrischen Farbmine mit einem Durchmesser von ca. 4,3 mm.

| Zusammensetzung der Minenmasse: | |
|---|---|
| Methylhydroxyethylcellulose | 4,0 g |
| feinvermahlenes Quarzmehl | 60,0 g |
| Bimsmehl | 60.0 g |
| Zinksulfid-/Bariumsulfatpigment (CAS Nr. 1345-05-07) | 10,0 g |
| feinvermahlenes Titandioxid (Rutil) | 13,0 g |
| Kupferphthalocyaninblau (Pigment Blue 15:3, C.I.Nr.74160) | 1,0 g |
| Ultramarinblau (Pigment Blue 29, C.I. Nr. 77007) | 8,0 g |
| Japanwachs | 2,0 g |
| Glyzerin | 2,0 g |

Die Masse wird in Analogie zu Beispiel 1 zu Rohminen verarbeitet. Die erhaltenen Minen zeigen einen hellblauen Abstrich auf Papier, bei befriedigender Minenmassenabgabe. Werden diese Minen bei 230° C 20 Minuten getempert, erhalten sie eine um etwas mehr als 20% reduzierte Bruchfestigkeit. Gleichzeitig zeigen diese Minen aber einen sehr weichen, guten Abstrich und eine ausgezeichnete Abgabe auf Papier.

### Beispiel 4:

Herstellung einer hautfarbigen Pudermine für Schmink- und Kosmetikanwendungen mit einem Durchmesser von ca. 4,5 mm.

| Zusammensetzung der Minenmasse: | |
|---|---|
| Hydroxyethylcellulose | 1,0 g |
| Methylhydroxyethylcellulose | 3,0 g |
| Talkum | 140,0 g |
| Magnesium Myristat | 6.0 g |
| Titandioxid | 40,0 g |
| Eisenoxid (Pigment Red 110 CAS-Nr. 1309-37-1) | 10,0 g |

Die Masse wird mit ca. 40 g Wasser homogen im Kneter vermischt und entsprechend Beispiel 1 zu Rohminen verarbeitet. Die so erhaltenen Minen werden zunächst 1 Stunde bei 230° C und dann 1 Stunde bei 240° C getempert. Es werden Minen mit einem puderförmigen Abstrich erhalten.

### Beispiel 5:

Herstellung einer fleischfarbenen puderförmigen Mine für Schmink- und Kosmetikzwecke mit einem Durchmesser von ca. 4,3 mm.

| Zusammensetzung der Minenmasse: | |
|---|---|
| Methylhydroxyethylcellulose | 20,0 g |
| Magnesium Myristat | 6,0 g |
| Titandioxid | 40,0 g |
| Glimmer | 140 g |
| Eisenoxid (Pigment Red 101 CAS-Nr. 1309-37-1) | 10,0 g |

Die pulverförmigen Ausgangsstoffe werden zunächst trocken, dann mit ca. 45 g Wasser homogen vermischt und wie in Beispiel 1 Rohminen extrudiert. Die so erhaltenen Rohminen werden 30 Minuten bei ca. 260° C gleichmäßig getempert. Es werden puderförmige zum Schminken geeignete Minen erhalten.

### Beispiel 6:

Herstellung einer pinkfarbenen Mine, die neben Anwendung auf Papier auch für Schminkzwecke geeignet ist. Durchmesser der Mine ca. 4,3 mm.

| Zusammensetzung der Minenmasse: | |
|---|---|
| Methylhydroxyethylcellulose | 3,8 g |
| Talkum | 140,4 g |
| Magnesium Myristat | 6,0 g |
| Titandioxid | 40,0 g |
| D + C-Red 7 (C.I. 15850) | 14,0 g |

Es werden zunächst analog Beispiel 1 Rohminen unter Zusatz von ca. 40 g Wasser hergestellt. Die Rohminen werden bei 245°C 30 Minuten lang getempert. Es werden Minen erhalten, die einen gleichmäßigen Abstrich auf Papier zeigen und auch zum Bemalen von Haut (Schminkanwendung) geeignet sind.

## Patentansprüche

1. Mine für Farbstifte, Kosmetikstifte und Farbkreiden,
dadurch gekennzeichnet, daß sie ein durch eine thermische Behandlung teilweise zersetztes polymeres Bindemittel enthält.

2. Mine nach Anspruch 1,
dadurch gekennzeichnet, daß das Bindemittel ein Biopolymer aus der Gruppe der Cellulosederivate und Alginate ist.

3. Mine nach Anspruch 2,
gekennzeichnet durch folgende Zusammensetzung (Gew.%):
| | |
|---|---|
| Bindemittel | 0,5 - 12,5% |
| Füllstoffe | 30 - 90% |
| Farbmittel | 5 - 96% |
| Wachse, Fette,Fettsäuren und/oder Emulgatoren | 0 - 25% |
| Hilfsstoffe | 0 - 10% |
| Wasser | < 1% |

4. Mine nach Anspruch 3,
dadurch gekennzeichnet, daß als Bindemittel wenigstens ein Stoff aus der Gruppe Alkalicarboxymethylcellulose, Ammonium-Carboxymethylcellulose, Hydroxyethylcellulose und Methylhydroxyethylcellulose ausgewählt ist.

5. Mine nach Anspruch 3 oder 4,
dadurch gekennzeichnet, daß sie wenigstens einen Füllstoff aus der Gruppe natürliche Magnesiumsilikate, Kaolin, Quarzmehl, Bimsmehl, Talkum, vermahlener Ton und Glimmer enthält.

6. Mine nach einem der Ansprüche 3 bis 5,
gekennzeichnet durch Farbmittel aus der Gruppe anorganische oder organische Pigmente, Farbstoffe, verlackte Farbstoffe, Metallbronzen, beschichtete Metallbronzen.

7. Mine nach einem der Ansprüche 3 bis 6,
dadurch gekennzeichnet, daß als Hilfsstoffe Tenside, Glykole, Alkohole, Fette, Fettsäuren, Wachse und/oder Emulgatoren enthalten sind.

8. Verfahren zur Herstellung einer Mine nach den Ansprüche 1-7,
mit folgenden Verfahrensschritten:
a) Die zerkleinerten, etwa pulver- oder granulatförmigen, ein thermolabiles polymeres Bindemittel, anorganische Füllstoffe und Farbmittel enthaltenden Ausgangsstoffe werden mit Wasser vermischt,
b) die so erhaltene Rohmasse wird zu Rohminen extrudiert,
c) die Rohminen werden bei einer erhöhten Temperatur, einer Zersetzungstemperatur, solange wärmebehandelt, bis sich das Bindemittel teilweise zersetzt hat.

9. Verfahren nach Anspruch 8,
dadurch gekennzeichnet, daß als Bindemittel Cellulosederivate oder Alginate eingesetzt und die Rohminen bei einer Temperatur von 150° C bis 350°C wärmebehandelt werden.

10. Verfahren nach Anspruch 9,
dadurch gekennzeichnet, daß als Bindemittel Alkalicarboxymethylcellulose, Ammonium-Carboxymethylcellulose, Hydroxyethylcellulose, Methylhydroxyethylcellulose und/oder Alginate verwendet werden.

11. Verfahren nach einem der Ansprüche 8 bis 10,
gekennzeichnet durch eine Wärmebehandlung mit einer Dauer von 10 Minuten bis 24 Stunden.

12. Verfahren nach einem der Ansprüche 8 bis 11,
dadurch gekennzeichnet, daß die Rohminen vor Verfahrensschritt c) getrocknet werden.

13. Verfahren nach Anspruch 12,
gekennzeichnet durch eine Trockentemperatur von 30°C bis 110° C.

14. Verfahren nach einem der Ansprüche 8 bis 13,
gekennzeichnet durch eine während der im wesentlichen gesamten Dauer der Wärmebehandlung im wesentlichen gleichbleibenden Zersetzungstemperatur.

15. Verfahren nach einem der Ansprüche 8 bis 13,
gekennzeichnet durch eine stufige Temperaturführung bei der Wärmebehandlung, wobei in der ersten Stufe eine niedrigere und in der zweiten Stufe eine höhere Zersetzungstemperatur angewendet wird.

16. Verfahren nach einem der Ansprüche 8 bis 15,
dadurch gekennzeichnet, daß die wärmebehandelten Minen durch Eintauchen in flüssige Fette, Wachse, Fettsäuren und/oder Emulgatoren mit diesen Stoffen getränkt werden.

17. Verfahren nach einem der Ansprüche 8 bis 15,
dadurch gekennzeichnet, daß den Ausgangsstoffen Fette, Wachse, Fettsäuren und/oder Emulgatoren beigemengt werden.

18. Verfahren nach Anspruch 17,
dadurch gekennzeichnet, daß bis 2 Gew.% Wachse, Fette, Fettsäuren und/oder Emulgaroen beigemengt werden.

## Claims

1. Lead for coloured pencils, cosmetics pencils and coloured chalks, characterized in that it comprises a polymeric binder partly decomposed by a heat treatment.

2. Lead according to Claim 1, characterized in that the binder is a biopolymer from the group consisting of cellulose derivatives and alginates.

3. Lead according to Claim 2, characterized by the following composition (% by weight):
| | |
|---|---|
| Binder | 0.5 - 12.5% |
| Fillers | 30 - 90% |
| Colorants | 5 - 96% |
| Waxes, fats, fatty acids | |
| and/or emulsifiers | 0 - 25% |
| Auxiliaries | 0 - 10% |
| Water | < 1% |

4. Lead according to Claim 3, characterized in that at least one substance from the group consisting of alkali metal carboxymethylcellulose, ammonium carboxymethylcellulose, hydroxyethylcellulose and methylhydroxyethylcellulose is chosen as the binder.

5. Lead according to Claim 3 or 4, characterized in that it comprises at least one filler from the group consisting of naturally occurring magnesium silicates, kaolin, ground quartz, ground pumice, talc, ground clay and mica.

6. Lead according to one of Claims 3 to 5, characterized by colorants from the group consisting of inorganic or organic pigments, dyestuffs, laked dyestuffs, metal bronzes and coated bronzes.

7. Lead according to one of Claims 3 to 6, characterized in that it comprises surfactants, glycols, alcohols, fats, fatty acids, waxes and/or emulsifiers as auxiliaries.

8. Process for the preparation of a lead according to Claims 1 - 7 with the following process steps:
a) the comminuted, such as pulverulent or granular, starting substances comprising a thermolabile polymeric binder, inorganic fillers and colorants are mixed with water,
b) the crude composition thus obtained is extruded to crude leads,
c) the crude leads are heat-treated at an elevated temperature, a decomposition temperature, until the binder has partly decomposed.

9. Process according to Claim 8, characterized in that cellulose derivatives or alginates are employed as the binder and the crude leads are heat-treated at a temperature from 150°C to 350°C.

10. Process according to Claim 9, characterized in that alkali metal carboxymethylcellulose, ammonium carboxymethylcellulose, hydroxyethylcellulose, methylhydroxyethylcellulose and/or alginates are used as the binder.

11. Process according to one of Claims 8 to 10, characterized by a heat treatment with a duration of 10 minutes to 24 hours.

12. Process according to one of Claims 8 to 11, characterized in that the crude leads are dried before process step c).

13. Process according to Claim 12, characterized by a drying temperature from 30°C to 110°C.

14. Process according to one of Claims 8 to 13, characterized by a decomposition temperature which is essentially constant during substantially the total duration of the heat treatment.

15. Process according to one of Claims 8 to 13, characterized by a stepwise temperature programme during the heat treatment, a lower decomposition temperature in the first stage and a higher decomposition temperature in the second stage being applied.

16. Process according to one of Claims 8 to 15, characterized in that the heat-treated leads are impregnated with liquid fats, waxes, fatty acids and/or emulsifiers by immersion in these substances.

17. Process according to one of Claims 8 to 15, characterized in that fats, waxes, fatty acids and/or emulsifiers are admixed to the starting substances.

18. Process according to Claim 17, characterized in that up to 2% by weight of waxes, fats, fatty acids and/or emulsifiers are admixed.

## Revendications

1. Mine pour crayons de couleur, crayons cosmétiques et craies de couleur, caractérisée en ce qu'elle contient un liant polymère se décomposant partiellement par un traitement thermique.

2. Mine selon la revendication 1, caractérisée en ce que le liant est un biopolymère du groupe des dérivés de la cellulose et des alginates.

3. Mine selon la revendication 2, caractérisée par la composition suivante (% en poids):
| | |
|---|---|
| Liant | 0,5 - 12,5% |
| Charge | 30 - 90% |
| Colorant | 5 - 96% |
| Cires, graisses, acides gras et/ou émulsionnants | 0 - 25% |
| Auxiliaires | 0 - 10% |
| Eau | <1% |

4. Mine selon la revendication 3, caractérisée en ce que l'on choisit comme liant au moins une matière parmi une alcalicarboxyméthylcellulose, l'ammonium-carboxyméthylcellulose, l'hydroxyéthylcellulose et la méthylhydroxyéthylcellulose.

5. Mine selon la revendication 3 ou 4, caractérisée en ce qu'elle contient au moins une charge parmi des silicates de magnésium naturels, du kaolin, de la poudre de quartz, de la poudre de pierre ponce, du talc, de l'argile ou du mica broyés.

6. Mine selon l'une quelconque des revendications 3 à 5, caractérisée par un colorant du groupe des pigments inorganiques ou organiques, des colorants, des colorants laqués, des bronzes métalliques, des bronzes métalliques revêtus.

7. Mine selon l'une quelconque des revendications 3 à 6, caractérisée en ce que sont contenus comme auxiliaires des tensioactifs, des glycols, des alcools, des graisses, des acides gras, des cires et/ou des émulsionnants.

8. Procédé pour la préparation d'une mine selon les revendications 1-7 avec les étapes suivantes du procédé :
a) on mélange avec de l'eau les matières premières broyées, à peu près dans la forme d'une poudre ou de granulés, contenant un liant polymère thermiquement instable, des charges inorganiques et un colorant,
b) on extrude la masse brute ainsi obtenue en des mines brutes,
c) on traite thermiquement les mines brutes à une température élevée, une température de décomposition jusqu'à ce que le liant s'est partiellement décomposé.

9. Procédé selon la revendication 8, caractérisé en ce que l'on utilise comme liants des dérivés de la cellulose ou des alginates et on traite thermiquement les mines brutes à une température de 150°C à 350°C.

10. Procédé selon la revendication 9, caractérisé en ce que l'on utilise comme liant une alcalicarboxyméthylcellulose, l'ammonium-carboxyméthylcellulose, l'hydroxyéthylcellulose, la méthylhydroxyéthylcellulose et/ou des alginates.

11. Procédé selon l'une quelconque des revendications 8 à 10, caractérisé par un traitement thermique avec une durée de 10 minutes à 24 heures.

12. Procédé selon l'une quelconque des revendications 8 à 11, caractérisé en ce que l'on sèche les mines brutes avant l'étape c) du procédé.

13. Procédé selon la revendication 12, caractérisé par une température de séchage de 30°C à 110°C.

14. Procédé selon l'une quelconque des revendications 8 à 13, caractérisé par une température de décomposition pratiquement constante pendant pratiquement la durée totale du traitement thermique.

15. Procédé selon l'une quelconque des revendications 8 à 13, caractérisé par une réalisation en étapes de la température lors du traitement thermique, une température de décomposition plus faible étant appliquée dans la première étape et une température de décomposition plus élevée étant appliquée dans la seconde étape.

16. Procédé selon l'une quelconque des revendications 8 à 15, caractérisé en ce que les mines traitées thermiquement sont, par immersion dans des graisses, des cires, des acides gras et/ou des émulsionnants liquides, imprégnées avec ces matières.

17. Procédé selon l'une quelconque des revendications 8 à 15, caractérisé en ce que l'on mélange aux matières premières des graisses, des cires, des acides gras et/ou des émulsionnants.

18. Procédé selon la revendication 17, caractérisé en ce que l'on mélange jusqu'à 2% en poids de cires, de graisses, d'acides gras et/ou d'émulsionnants.
